# EUROPEAN PATENT APPLICATION

(11) **EP 4 356 757 A1**
(43) Date of publication of application: **24.04.2024**
(21) Application number: 22382999.5
(22) Date of filing: 18.10.2022
(51) Int. Cl.: A23L 33/135, A61P 3/00, A61K 31/366, C07D 405/12, C12N 1/20, C12P 7/22, C12P 17/06, C12P 39/00, C12R 1/01, C12R 1/145

(54) **MICROBIAL METHOD FOR REPRODUCING THE HUMAN UROLITHIN METABOTYPES IN VITRO AND IN VIVO**

(71) Applicant: Consejo Superior de Investigaciones Científicas (CSIC), 28006 Madrid (ES)
(72) Inventor: Selma García, María Victoria, Madrid (ES); Tomás-Barberán, Francisco A., Madrid (ES); Beltrán Riquelme, David, Madrid (ES); Espín de Gea, Juan Carlos, Madrid (ES); García Villalba, Rocío, Madrid (ES)
(74) Representative: Pons

(57) **Abstract**

The present invention relates to a new bacterial strain and a new urolithin named urolithin G. The new strain of the invention is *Enterocloster bolteae* CEBAS S4A9 DSM 34392 and can customize urolithin production to mimic metabotype A and metabotype B *in vitro* and *in vivo* if combined with at least one bacterial strain belonging to the *Gordonibacter* and/or *Ellagibacter* and/or *Slackia* genera. The invention also refers to methods or uses of *E. bolteae* CEBAS S4A9 DSM 34392 or the consortium that comprises *E. bolteae* CEBAS S4A9 DSM 34392 and at least one bacterial strain belonging to the *Gordonibacter* and/or *Ellagibacter* and/or *Slackia* genera for producing urolithins. The invention also refers to compositions or food products that contain *E. bolteae* CEBAS S4A9 DSM 34392, the aforementioned consortium, or the new urolithin G.

## Description

The present invention is within the biotechnological sector. The present invention relates to the use of enteric bacteria to produce urolithins. More particularly, the present invention refers to the use of bacteria belonging to the *Enterocloster* genus, either alone or in combination with other enteric bacteria belonging to the *Gordonibacter* and/or *Ellagibacter* and/or *Slackia* genera, to customize urolithin production to mimic the human metabotype A and metabotype B *in vitro* and *in vivo.*

### BACKGROUND ART

The consumption of ellagitannins (ETs) and ellagic acid (EA) rich foods, such as nuts, pomegranates, berries, and the like, has been associated with beneficial health effects. EA is released through the hydrolysis of ET's ester bonds by the enzyme known as ellagitannase. Consequently, EA undergoes further degradation, which leads to the production of 6H-dibenzo[*b,d*]pyran-6-one derivatives named Urolithins (Uros). The human gut microbiota is responsible for converting these polyphenols into Uros, which show anti-inflammatory, antioxidant, anticarcinogenic, cardioprotective, and neuroprotective effects.

This cascade of reactions starts with a lactone ring cleavage by a lactonase enzyme, resulting in luteic acid, which is then decarboxylated to pentahydroxy-Uro (Uro-M5). Next, consecutive dehydroxylations convert it to tetrahydroxy-Uros (Uro-D, Uro-E, and Uro-M6), trihydroxy-Uros (Uro-C and Uro-M7) to finally obtain dihydroxy-Uros (Uro-A and isoUro-A) and monohydroxy-Uro (Uro-B). This latter Uro is generally detected when isoUro-A is also produced.

Urolithins (Uros) have gained recognition as one of the main drivers of the health effects related to the intake of ellagitannins (ETs) and ellagic acid (EA) rich foods. However, Uros production capacity and, consequently, at least partly, the health effects associated with ETs consumption vary among individuals because not all individuals have the gut bacteria needed to produce all the Uros (Cortés-Martín et al., 2020b; Iglesias-Aguirre et al., 2021).

According to how ETs are metabolized by the gut microbiota, individuals can be stratified into urolithin metabotypes (UMs) associated with particular gut microbiome compositions and functionalities. Three human UMs (UM-A, UM-B, and UM-0) are related to three different Uros production profiles. Metabotype A (UM-A) individuals produce several intermediate Uros but only urolithin A (Uro-A) at the end of the microbial catabolic pathway, being Uro-A the most absorbed Uro in the intestine. Metabotype B (UM-B) individuals produce a different urolithin profile, including some intermediate Uros and three final Uros, i.e., urolithin B (Uro-B), isourolithin A (IsoUro-A) and Uro-A, which are the mainly absorbed Uros in this metabotype. Metabotype 0 (UM-0) individuals cannot produce Uros (only the precursor Urolithin-M5 has been detected so far, which is not absorbed in the gut). One of the differences between the metabolic profiles associated with UMs is the final Uros produced. Therefore, UM-A individuals only produce Uro-A as the final metabolite of the EA metabolic pathway, whereas UM-B subjects produce Uro-A and, distinctively, IsoUro-A and Uro-B. Finally, UM-0 individuals cannot produce Uros (only the precursor Uro-M5 has been detected so far).

Therefore, there is an interest in elucidating the bacteria responsible for the complete catabolism of ellagic acid and ellagitannins into the full profile of urolithins associated with metabotype A and metabotype B. The bacterial genera *Gordonibacter* and *Ellagibacter* have been recently identified as capable of metabolizing EA into some Uros *in vitro* (Selma et al., Food Funct., 2014, 5, 1779; Selma et al., Front. Microbiol., 2017, 8, 1-8). However, some intermediates (for example, Uro-D, Uro-E, Uro-M7 ) and final Uros (Uro-A and Uro-B) were not produced in pure cultures of these bacteria, which implies the need for other bacteria to complete the Uro profile that configures both UM-A and UM-B. However, the bacterial consortia capable of reproducing human urolithin profiles associated with UM-A and UM-B *in vivo* is still unknown.

Since urolithins are bioactive compounds with some benefits for healthcare, it is of interest to develop new strategies and methods to produce and use them in the pharma, cosmetic, and food industry. In addition, strategies to convert non-urolithin-producing individuals into urolithin producers, characteristic of UM-A and UM-B, are also of interest.

The document US2016/0332982 describes the chemical synthesis of Uro-A and its use for treating neurological diseases. However, the compounds obtained by chemical synthesis are not usually well accepted by public opinion.

The document US2019/0323045 discloses a biological method to produce urolithin E, urolithin M7, urolithin A, and urolithin B starting from urolithins having a hydroxyl group at the 9-position, which is eliminated by the action of a bacteria belonging to the *Clostridium* genus.

However, none of the cited documents describes the complete production of the mix of urolithins that conform the metabotypes A or B.

Moreover, urolithins have proved their efficacy in treating various diseases or pathological conditions. EP2068864B1 describes the use of urolithins for the treatment of cancer. EP3278800B1 describes the use of urolithins for the treatment of muscle diseases. EP2654461B1 describes the use of urolithins for the treatment of metabolic diseases. Finally, EP3393467B1 describes the use of urolithins for treating neurodegenerative and mitochondrial diseases. However, the cited documents just refer to the already-known urolithins' medical uses. This group of metabolites continuously grows by discovering new urolithins that may have the same therapeutic potential.

Therefore, in view of the cited prior art, as the urolithins, which are comprised of metabotypes A and B, have several health benefits, there is a need in the prior art to provide new natural and well-accepted strategies that allow producing urolithins *in vitro,* or *in vivo* by the administration of urolithin-producing bacteria in the form of probiotic or postbiotic compositions. Specifically, it is interesting to discover and provide bacterial consortia capable of reproducing human urolithin profiles associated with UM-A and UM-B, wherein said bacterial consortia could be used to convert non-urolithin-producing individuals into producers of the characteristic urolithins of UM-A and UM-B when administered to said individuals, preferably humans, but also animals. The discovery and characterization of new urolithins with health effects would also provide new molecules that can be used for health benefits.

### DESCRIPTION OF THE INVENTION

The present invention discloses a novel bacteria strain belonging to the *Enterocloster* genus that produces urolithins and novel gut bacterial consortia comprising said bacteria and other bacteria belonging to the *Ellagibacter* and/or *Gordonibacter* and/or *Slackia* genera wherein the consortia produce the mix of urolithins of metabotypes A or B either *in vitro* or *in vivo,* as well as a newly characterized urolithin.

The novel bacteria belonging to the *Enterocloster* genus is CEBAS S4A9 strain, identified as belonging to the species *Enterocloster bolteae* with the deposit number DSM 34392 (*Enterocloster bolteae* CEBAS S4A9 = DSM 34392), isolated from the feces of a healthy woman, capable of producing the final metabolites Uro-A and Uro-B, but also the different intermediates such as Uro-D, Uro-E, Uro-M7 and the novel urolithin-G (Uro-G), confirmed by NMR analysis. Together with this strain CEBAS S4A9, the inventors showed that other strains and species of the same bacterial genus (*Enterocloster*) also catalyze the dehydroxylation of hydroxyl groups at positions 9 and 10, regardless of whether they are in a catechol ring or not.

The gut bacterial consortia found by the inventors reproduce the urolithin synthesis pathways associated with UM-A and UM-B upon *in vitro* fermentation. The inventors also found that these consortia colonized the intestine and converted UM-0 rats into Uro-producers that mimicked UM-A and UM-B, respectively. Therefore, the present invention also refers to the use of the consortia that comprises *E. bolteae* CEBAS S4A9 DSM 34392 strain in combination with other enteric bacteria belonging to the *Gordonibacter* and/or *Ellagibacter* and/or *Slackia* genera to customize urolithin production to mimic metabotype A and metabotype B *in vitro* and *in vivo.* The term "customize" refers to changing urolithin production based on the needs of each individual.

The urolithins-producing strain *E. bolteae* CEBAS S4A9 DSM 34392, and the bacterial consortia that comprise it, could have potential as novel probiotics and postbiotics in the industrial manufacture of bioactive urolithins to develop new ingredients, beverages, nutraceuticals, pharmaceuticals, and/or functional foods. This is especially relevant in UM-0 individuals.

The new urolithin of the present invention is named urolithin G (Uro-G), which corresponds to 3,4,8-trihydroxy urolithin. The inventors found that urolithin G is produced by bacteria belonging to the *Enterocloster* genus. Urolithin G showed a ¹H NMR (500 MHz in deuterated acetonitrile: ACN-d3) with chemical shift values (δ) in ppm: 7.48 (d, 1H, J_{H1-H2} = 8.2 Hz, H-1); 6.82 (d, 1H, J_{H2-H1} = 8.20 Hz, H-2); 7.61 (d, 1H, J_{H7-H-9} =2.2 Hz, H-7); 7.33 (dd, 1H, J_{H9-H10} = 8.53 Hz, J_{H9-H7} = 2.2 Hz, H-9); 8.03 (d, H, J_{H10-H9}=8.5 Hz, H-10).

The urolithin G is represented by the structure (I):

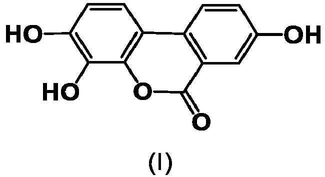

The urolithin G was structurally characterized by having the same common structure, and therefore similar properties, of the already known urolithins. The common structure of the urolithins corresponds to the structure (II):

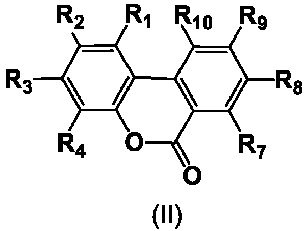

Wherein the R1 to R8 each represents a hydroxyl group, a hydrogen atom, or a methoxy group, and at least one of R1 to R8 represents a hydroxy group.

Examples of urolithins are represented by the following structures:

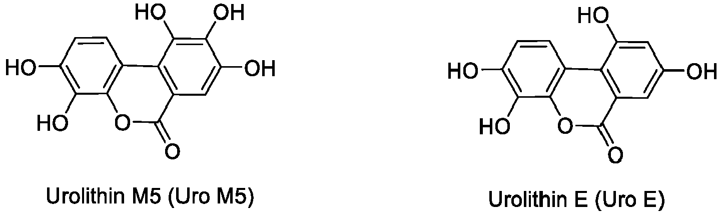

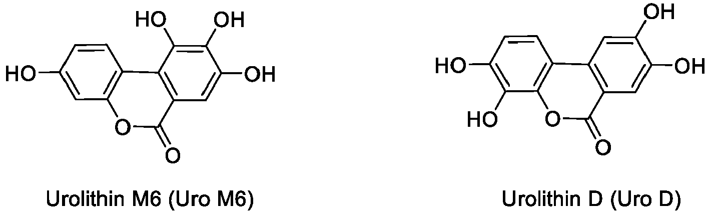

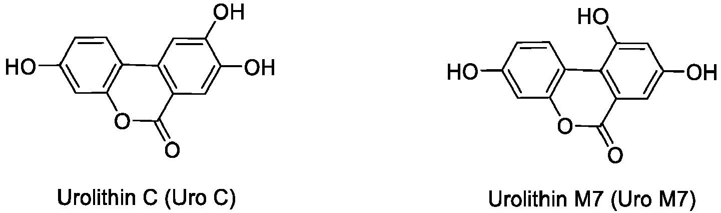

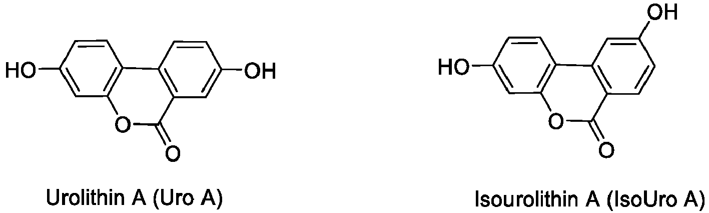

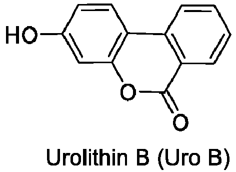

In one aspect, the present invention relates to a bacterial strain belonging to the *Enterocloster* genus that produces urolithins, wherein said strain is *Enterocloster bolteae* CEBAS S4A9 with deposit number DSM 34392 in the form of a living or an inactivated cell, hereinafter referred to as "the strain of the invention".

The strain of the invention may be named "CEBAS S4A9 strain", "*Enterocloster bolteae* CEBAS S4A9 with the deposit number DSM 34392", "*Enterocloster bolteae* CEBAS DSM 34392" or "*Enterocloster bolteae* DSM 34392". The strain *E. bolteae* CEBAS S4A9 was received on September 26th, 2022, for patent deposit according to the Budapest Treaty, at DSMZ - Deutsche Sammlung von Mikroorganismen und Zellkulturen GmbH (Leibniz Institute DSMZ-German Collection of Microorganisms and Cell Cultures; address Inhoffenstr. 7B D-38124 Braunschweig); the depositor being "Consejo Superior de Investigaciones Científicas" (address Calle Serrano 142, 28006 Madrid Spain).

The term "inactivated", as used in the description, refers to the characteristic of a bacterium that cannot grow after undergoing a process of inactivation, where the bacterium retains its structure. Methods for inactivating bacteria are known for a skilled person in the art, being some examples, but not limited to, the use of chemical agents (i.e., glutaraldehyde, formalin, or paraformaldehyde), and the application of heat, high pressure or UV irradiation. In contrast, a "living" bacterium can grow.

In the context of the present invention, the strain of the invention can be present as the wild-type strain of the invention or as the mutant strain of the invention. The term wild-type refers to a strain having a phenotype, genotype, or gene that predominates in a natural population of organisms. The term "mutant" or "derived" refers to the strain of the invention comprising at least one variation or mutation in its genome, wherein the variation in the genome of the derived or mutant strain of the invention is not present in the genome of the wild-type strain of the invention. The variation in the genome may be a point mutation, an insertion, a deletion, or combinations thereof, wherein the variation or variations in the genome of the mutant strain of the invention do not affect the cell viability. Thus, the strain of the invention can be as the wild-type strain of the invention in the form of a living or inactivated cell or as the mutant strain of the invention in the form of a living or inactivated cell.

In another aspect, the invention relates to a culture, a lysate, or a culture supernatant of the bacterial strain of the invention, present as the wild-type or mutant strain of the invention.

The term "culture", as used in the description, refers to a composition that comprises bacteria and a culture medium that contains all the minimal nutritional elements that allow the growth of the bacteria that are present in it. Some examples of culture media are, but not limited to, Anaerobe Basa Broth (ABB medium) or Wilkins-Chalgren Anaerobe Broth.

The term "lysate" refers to a composition comprising the components of a bacterial cell, such as cell membrane fragments and molecules from the content of bacteria, such as enzymes or DNA, that are released after the lysis process. The lysis of bacteria can be carried out by any technique or method known in the art, such as, but not limited to, mechanical lysis (by grinding the bacteria), sonication, freeze-thaw cycles, or liquid homogenization with a lysis buffer.

The term "culture supernatant" refers to the liquid fraction of a liquid culture medium where the bacteria were grown, where the liquid fraction is separated from the bacteria by any means like centrifugation or filtration.

The inventors found that the strain of the invention produced urolithins starting from other urolithins as raw material when cultured alone. More specifically, the strain of the invention produced urolithin D starting from urolithin M5, urolithin E starting from urolithin M5, urolithin M7 starting from urolithin M6, urolithin G starting from urolithin D, urolithin A starting from urolithin C and urolithin B starting from isourolithin A.

As used in the present invention the terms "raw material" or "precursor", used interchangeably, refer to a substance from which another is formed, especially by metabolic reaction.

In another aspect, the invention relates to an *in vitro* use of the strain of the invention in the form of a living or an inactivated cell or the culture, lysate, or culture supernatant of the strain of the invention to produce urolithins, preferably the production of urolithins is carried out starting from a first urolithin as raw material.

In the context of the present invention, "first urolithin" refers to a urolithin as the substrate of the reaction that leads to the production of a second urolithin, wherein the second urolithin can also be referred to as "a produced urolithin".

In a preferred embodiment, the first urolithin is selected from the list consisting of urolithin M7, urolithin M6, urolithin D, urolithin C, and isourolithin A, wherein the first urolithin is metabolized to produce a second urolithin by the strain of the invention.

In a preferred embodiment, the first urolithin is urolithin M5, and the produced urolithin is urolithin D or E; or the first urolithin is urolithin M6, and the produced urolithin is urolithin M7; or the first urolithin is urolithin D, and the produced urolithin is urolithin G; or the first urolithin is urolithin C, and the produced urolithin is urolithin A, or the first urolithin is isourolithin-A, and the produced urolithin is urolithin B; wherein the urolithin G is a urolithin represented by the formula (I):

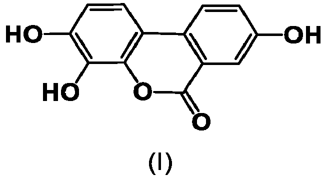

In another aspect, the present invention refers to a method for producing urolithins wherein the production of urolithins is carried out starting from a first urolithin as raw material, wherein said method comprises the following steps:
a) Adding the first urolithin as a raw material of a second urolithin into a solution containing the strain of the invention in the form of a living or an inactivated cell, a culture, a lysate, or culture supernatant of the strain of the invention.
b) Converting the first urolithin to a second urolithin with the strain of the invention, a culture of the strain of the invention, a lysate of the strain of the invention, or culture supernatant of the strain of the invention.

In a preferred embodiment, the first urolithin is M5, and the produced urolithin is D; or the first urolithin is M5, and the produced urolithin is E; or the first urolithin is M6, and the produced urolithin is M7; or the first urolithin is D, and the produced urolithin is G; or the first urolithin is C, and the produced urolithin is A; or the first urolithin is isourolithin-A, and the produced urolithin is urolithin B.

In a preferred embodiment, the method of producing urolithins further comprises a step c): purifying and/or concentrating the second urolithin obtained by step b).

As used in the present description, the term "purification" refers to a treatment such as sterilization of the microorganism by heat or the like; elimination of the microorganism by microfiltration (MF), ultrafiltration (UF), or the like; removal of solid matters and macromolecular substances; extraction with an organic solvent or an ionic liquid; or adsorption or decoloration using a hydrophobic adsorbent, ion exchange resin, activated carbon column, or the like.

As used in the present description, the term "concentration" refers to a known method for the skilled person, including concentration using an evaporator, reverse osmosis membrane, or the like.

The solution containing the strain of the invention, a culture of the strain of the invention in the form of a living or an inactivated cell, a lysate of the strain of the invention, or culture supernatant of the strain of the invention is not limited as long as the ability to produce the second urolithin from the first urolithin can be allowed to produce the second urolithin from the first urolithin in the solution.

As used in the present description, the term "solution" refers to a medium for culturing bacteria. The medium is not limited, and examples of the medium include Anaerobe Basa Broth (ABB medium), manufactured by Oxoid Limited; Wilkins-Chalgren Anaerobe Broth (CM0643), manufactured by Oxoid Limited; and GAM medium and modified GAM medium, manufactured by Nissui Pharmaceutical Co., Ltd. The "solution" can also be a buffer solution wherein some examples of buffers are, but with no limit to, phosphate buffer saline (PBS), phosphate buffer, pyrophosphate buffer, carbonate buffer, borate buffer, or formate buffer, acetate buffer, tartrate buffer, tris or organic citrate buffer. Bacteria can be first cultured and then placed in a buffer solution to produce urolithins.

In a preferred embodiment, the content of the first urolithin in the solution is usually not less than 0.01 g/L, preferably not less than 0.1 g/L, more preferably not less than 1.0 g/L. On the other hand, the content is usually not more than 100 g/L, preferably not more than 20 g/L, more preferably not more than 10 g/L.

In a preferred embodiment, the amount of strain of the invention is not less than 10³ CFU/mL in the solution of step a); preferably not less than 10⁶ CFU/mL in the solution of step a). In a more preferred embodiment, the amount of strain of the invention in the solution of step a) is between 10³ and 10¹² CFU/mL; more preferably, the amount of strain of the invention in the solution of step a) is between 10⁶ and 10¹² CFU/mL; more preferably the amount of strain of the invention in the solution of step a) is between 10⁶ and 10¹⁰ CFU/mL

For the conversion of the first urolithin in step b), the temperature is set at a value between 20 to 50 ºC, preferably between 25 to 45 ºC, and more preferably at 37 ºC. The incubation time is at least 1 hour, preferably at least 24 h, and preferably at least 1 week.

The inventors found that the strain of the invention did not metabolize ellagic acid into other urolithins by itself and then did not produce the urolithin profiles of the human metabotypes A or B.

As used in the description, the term "metabotype" refers to a specific group of individuals, preferably humans, defined on the basis of their similarities in a metabolic profile. The term "metabotype A", "metabolic phenotype A", "urolithin metabotype A" or "UM-A", used interchangeably, refers to the group of individuals that produce the profile of urolithins that comprises, preferably consisting of Urolithin M6, urolithin D, urolithin E, urolithin C, urolithin M7 and urolithin A, and preferably the "urolithin metabotype A" further comprises the urolithin G represented by the formula (I). The term "metabotype B", "metabolic phenotype B", "urolithin metabotype B" or "UM-B" , used interchangeably, refers to the group of individuals that produce the profile of urolithins that comprises the urolithins of the metabotype A and further comprises isourolithin A and urolithin B. The term "metabotype 0", "metabolic phenotype 0" or "UM-0", used interchangeably, refers to the group of individuals that cannot produce the profile of metabotype A or B. Metabotype 0 individuals just metabolize or convert ellagic acid to urolithin M5, but urolithin M5 is not further converted into other urolithins like urolithin D, urolithin M6, or urolithin E. Therefore, the term "metabotype 0" refers to human individuals and/or animals incapable of producing urolithins, preferably the urolithins comprised in the metabotype A or B profile. In the present invention, the definition "human individuals and/or animals with the incapacity of producing urolithins" refers to human individuals and/or animals that metabolize ellagic acid to urolithin M5, but cannot further metabolize urolithin M5 into urolithins of the metabotype A or B (urolithin M5 is the precursor of the rest of the urolithins comprised in the profiles of metabotypes A or B). Specifically, these individuals do not metabolize urolithin M5 into urolithin D, urolithin M6, or urolithin E. In a preferred embodiment, the animals are mammals, preferably non-human mammals. In a more preferred embodiment, some examples of animals are, but not limited to, dogs, cats, horses, rabbits, cows, or pigs.

When the inventors combined the strain of the invention with a bacterium belonging to the *Gordonibacter* or *Ellagibacter* genera, the *in vitro* production of urolithins of the metabotype A or B, starting from ellagic acid, was observed. Specifically, the consortium comprising the strain of the invention with a bacterium belonging to the *Gordonibacter* genus showed the *in vitro* production of the urolithin profile of metabotype A. The consortium comprising the strain of the invention with bacterium belonging to the *Ellagibacter* genus showed the *in vitro* production of the urolithin profile of metabotype B. The consortium comprising the strain of the invention with bacterium belonging to the *Gordonibacter* and/or *Ellagibacter* genera combined or not with the *Slackia* genera showed the *in vitro* production of the urolithin profile of metabotype B. When the aforementioned consortia were administered to rats of metabotype 0, they acquired the capacity to produce the urolithins of metabotype A or B.

In another aspect, the invention relates to a urolithin-producing bacterial consortium, hereinafter referred to as "the bacterial consortium of the invention" or "consortium of the invention", used interchangeably, that comprises:
a. The strain of the invention in the form of a living or an inactivated cell, or the culture, lysate, or culture supernatant of the strain of the invention, and,
b. At least another living or inactivated bacterial strain belonging to the *Gordonibacter* and/or *Ellagibacter* and/or *Slackia* genera; or a culture, lysate, or culture supernatant of a bacterium belonging to the *Gordonibacter* and/or *Ellagibacter* and/or *Slackia* genera.

In the preferred embodiment, the urolithin-producing bacterial consortium of the invention comprises a combination of the strain of the invention and a bacterial strain belonging to the *Gordonibacter* genus. In a further preferred embodiment, the urolithin-producing bacterial consortium of the invention comprises a combination of the strain of the invention and a bacterial strain belonging to *Ellagibacter.* In a further preferred embodiment, the urolithin-producing bacterial consortium of the invention comprises a combination of the strain of the invention, a bacterial strain belonging to the *Gordonibacter* genus, and a bacterial strain belonging to the *Ellagibacter* genus. In a further preferred embodiment, the urolithin-producing bacterial consortium of the invention comprises a combination of the strain of the invention, a bacterial strain belonging to the *Gordonibacter* genus, and a bacterial strain belonging to the *Slackia* genus. In a further preferred embodiment, the urolithin-producing bacterial consortium of the invention comprises a combination of the strain of the invention, a bacterial strain belonging to the *Gordonibacter* genus, a bacterial strain belonging to the *Ellagibacter* genus, and a bacterial strain belonging to the *Slackia* genus.

In a preferred embodiment, the bacterial strain belonging to the *Gordonibacter* genus is a bacterium belonging to *Gordonibacter urolithinfaciens*, *Gordonibacter pamelaeae*, and/or *Gordonibacter massiliensis*; preferably *Gordonibacter urolithinfaciens* DSM27213, *Gordonibacter pamelaeae* DSM19378, and/or *Gordonibacter* 28C.

The bacterial strain belonging to the *Gordonibacter* genus can produce urolithin M5 starting from ellagic acid as raw material, urolithin M6 starting from urolithin M5, urolithin C starting from urolithin M6, urolithin C starting from urolithin D, urolithin A starting from urolithin G and urolithin M7 starting from urolithin E. Therefore, the enzymatic or metabolic activity of the strain of the invention and a bacterial strain belonging to the genus *Gordonibacter* complement each other to produce the urolithins comprised in the urolithin profiles corresponding to the metabotype A.

In a preferred embodiment, the bacterial strain belonging to the *Ellagibacter* genus is a bacterium belonging to *Ellagibacter isourolithinifaciens*; preferably *Ellagibacter isourolithinifaciens* DSM 104140.

The bacterial strain belonging to the *Ellagibacter* genus can produce urolithin M5 starting from ellagic acid as raw material, urolithin M6 starting from urolithin M5, urolithin C starting from urolithin M6 and isourolithin A starting from urolithin C. Therefore, the enzymatic or metabolic activity of the strain of the invention and a bacterial strain belonging to the *Ellagibacter* genus complement each other to produce the urolithin profiles corresponding to metabotype B.

In a preferred embodiment, the bacterial strain belonging to the *Slackia* genus is a bacterium belonging to *Slackia heliotrinireducens;* preferably *Slackia heliotrinireducens* DSM 20476.

The bacterial strain belonging to *Slackia* can produce isourolithin A starting from urolithin C. Therefore, the strain of the invention can be combined with a bacterial strain belonging to the *Gordonibacter* genus and a bacterial strain belonging to the *Slackia* genus to produce the urolithin profiles corresponding to metabotype B.

Examples of the bacteria included in the consortium of the invention are not limited to the species or strains of *Gordonicater*, *Ellagibacter* or *Slackia* mentioned above. Therefore, other bacteria or strains belonging to *Gordonicater*, *Ellagibacter* or *Slackia* can be comprised in consortium of the invention as long as it preserves its capacity to reproduce the urolithin profiles of metabotypes A and/or B.

In another aspect, the invention relates to an *in vitro* use of the urolithin-producing bacterial consortium of the invention for the production of urolithins from raw material, preferably wherein the raw material comprises urolithin M5, ellagic acid, and/or ellagitannins. Preferably, the raw material that comprises urolithin M5 is a solution containing urolithin M5. Preferably, the raw material comprising ellagic acid and/or ellagitannins can be a solution containing ellagic acid and/or ellagitannins, or ellagic acid and/or ellagitannins-rich food or extract thereof or their more soluble derivatives by adding amino acids, emulsifying polymer or encapsulation into (nano)liposomes. Examples of ellagic acid and/or ellagitannins-rich food are, but not limited to, raspberry, blackberry, cloudberry, boysenberry, strawberry, walnut, pomegranate, and/or geranium.

In a preferred embodiment, the raw material water solubility can be improved by preparing a co-amorphous with amino acids, such as β-alanine, L-arginine, or others.

In a preferred embodiment, the produced urolithins belong to the metabotype A when the urolithin-producing bacterial consortium comprises a) the strain of the invention or the culture, lysate or culture supernatant of the strain of the invention, and b) at least a living or inactivated bacterial strain belonging to the *Gordonibacter* genus or a culture, lysate or culture supernatant of the bacterium belonging to the *Gordonibacter* genus, preferably *Gordonibacter urolithinfaciens*, *or Gordonibacter pamelaeae*, *or Gordonibacter massiliensis;* more preferably *Gordonibacter urolithinfaciens* DSM27213, *or Gordonibacter pamelaeae* DSM19378, *or Gordonibacter* 28C*;*wherein the produced urolithins of the metabotype A are urolithin D, urolithin E, urolithin M6, urolithin C, urolithin G, urolithin M7 and/or urolithin A.

In a preferred embodiment, the produced urolithins belong to the metabotype B when the urolithin-producing bacterial consortium comprises a) the strain of the invention or the culture, lysate or culture supernatant of the invention, and b) at least a living or inactivated bacterial strain belonging to the *Ellagibacter* genus or a culture, lysate or culture supernatant of the bacterium belonging to the *Ellagibacter* genus; preferably *Ellagibacter isourolithinifaciens;* more preferably *Ellagibacter isourolithinifaciens* DSM 104140; and optionally c) at least a living or inactivated bacterial strain belonging to the *Gordonibacter* genus or a culture, lysate or culture supernatant of these bacteria, preferably *Gordonibacter urolithinfaciens, or Gordonibacter pamelaeae, or Gordonibacter massiliensis;* more preferably *Gordonibacter urolithinfaciens* DSM27213, *or Gordonibacter pamelaeae* DSM19378*, or Gordonibacter* 28C; wherein the produced urolithins of the metabotype B are urolithin C, urolithin G, urolithin M7, isourolithin A, urolithin A and/or urolithin B.

In a preferred embodiment, the produced urolithins belong to the metabotype B when the urolithin producing bacterial consortium comprises a) the bacterial strain of the invention or the culture, lysate or culture supernatant of the strain of the invention, and b) at least a living or inactivated bacterial strain belonging to the *Gordonibacter* genus or a culture, lysate or culture supernatant of these bacteria, preferably *Gordonibacter urolithinfaciens, or Gordonibacter pamelaeae, or Gordonibacter massiliensis;* more preferably *Gordonibacter urolithinfaciens* DSM27213, *or Gordonibacter pamelaeae* DSM 19378, *or Gordonibacter* 28C, and c) at least a living or inactivated bacterial strain belonging to the *Slackia* genus or a culture, lysate or culture supernatant of the bacterium belonging to the *Slackia* genus, preferably the bacterial strain belonging to the *Slackia* genus is a bacterium belonging to *Slackia heliotrinireducens;* preferably *Slackia heliotrinireducens* DSM 20476, and optionally d) the urolithin-producing bacterial consortium further comprises at least a living or inactivated bacterial strain belonging to the *Ellagibacter* genus or a culture, lysate or culture supernatant of the bacterium belonging to the *Ellagibacter* genus; preferably *Ellagibacter isourolithinifaciens;* more preferably *Ellagibacter isourolithinifaciens* DSM 104140; wherein the produced urolithins of the metabotype B are urolithin C, urolithin G, urolithin M7, isouroulithin A, urolithin A and/or urolithin B.

In another aspect, the invention refers to a method for producing urolithins of metabotypes A and/or B, wherein said method comprises the following steps:
a) adding a raw material comprising urolithin M5, ellagic acid, and/or ellagitannins or more soluble derivates into a solution containing the urolithin-producing bacterial consortium of the invention at different relative concentrations or incubation times to replicate the different urolithin proportions in metabotype A and/or metabotype B;
b) converting the raw material with the urolithin bacterial consortium of the present invention to urolithins of metabotype A and/or B.

Wherein the produced urolithins belong to the metabotype A when the urolithin-producing bacterial consortium comprises a) the bacterial strain of the invention or the culture, lysate or culture supernatant of the strain of the invention, and b) at least a living or inactivated bacterial strain belonging to the *Gordonibacter* genus or a culture, lysate or culture supernatant of the bacterium belonging to the *Gordonibacter* genus; wherein the produced urolithins of the metabotype A are urolithin D, urolithin E, urolithin M6, urolithin C, urolithin G, urolithin M7 and/or urolithin A.

Or wherein the produced urolithins belong to the metabotype B when the urolithin-producing bacterial consortium a) the bacterial strain of the invention or the culture, lysate or culture supernatant of the strain of the invention, and b) at least a living or inactivated bacterial strain belonging to the *Ellagibacter* genus or a culture, lysate or culture supernatant of the bacterium belonging to the *Ellagibacter* genus; preferably *Ellagibacter isourolithinifaciens;* more preferably *Ellagibacter isourolithinifaciens* DSM 104140; and optionally c) at least a living or inactivated bacterial strain belonging to the *Gordonibacter* genus or a culture, lysate or culture supernatant of these bacteria, preferably *Gordonibacter urolithinfaciens, or Gordonibacter pamelaeae, or Gordonibacter massiliensis,* more preferably *Gordonibacter urolithinfaciens* DSM27213, *or Gordonibacter pamelaeae* DSM19378*, or Gordonibacter* 28C; wherein the produced urolithins of the metabotype B are urolithin C, urolithin G, urolithin M7, isourolithin A, urolithin A and/or urolithin B.

Or wherein, the produced urolithins belong to the metabotype B when the urolithin-producing bacterial consortium comprises a) the bacterial strain of the invention or the culture, lysate or culture supernatant of the strain of the invention, and b) at least a living or inactivated bacterial strain belonging to the *Gordonibacter* genus or a culture, lysate or culture supernatant of these bacteria, preferably *Gordonibacter urolithinfaciens, or Gordonibacter pamelaeae, or Gordonibacter massiliensis; more preferably* preferably *Gordonibacter urolithinfaciens* DSM27213, *or Gordonibacter pamelaeae* DSM19378*, or Gordonibacter* 28C and c) at least a living or inactivated bacterial strain belonging to the *Slackia* genus or a culture, lysate or culture supernatant of the bacterium belonging to the *Slackia* genus, preferably the bacterial strain belonging to the *Slackia* genus is a bacterium belonging to *Slackia heliotrinireducens;* preferably *Slackia heliotrinireducens* DSM 20476, and optionally d) the urolithin-producing bacterial consortium further comprises at least a living or inactivated bacterial strain belonging to the *Ellagibacter* genus or a culture, lysate or culture supernatant of the bacterium belonging to the *Ellagibacter* genus; preferably *Ellagibacter isourolithinifaciens;* more preferably *Ellagibacter isourolithinifaciens* DSM 104140; wherein the produced urolithins of the metabotype Bare urolithin C, urolithin G, urolithin M7, isourolithin A, urolithin A and/or urolithin B.

In a preferred embodiment, the method for producing urolithins of the metabotypes A and/or B further comprises step c): purifying and/or concentrating the produced urolithins obtained by step b).

In a preferred embodiment, the content of the urolithin M5, ellagic acid, and/or ellagitannins in the solution is usually not less than 0.01 g/L, preferably not less than 0.1 g/L, more preferably not less than 1.0 g/L. On the other hand, the content is usually not more than 100 g/L, preferably not more than 20 g/L, more preferably not more than 10 g/L.

As described before, the term "solution" is understood as a culture or a solution buffer, as defined in the previous method.

In a preferred embodiment, the amount of the strain of the invention in the solution of step a) is not less than 10³ CFU/mL; preferably not less than 10⁶ CFU/mL. In a more preferred embodiment, the amount of strain of the invention in the solution of step a) is between 10³ and 10¹² CFU/mL; more preferably, the amount of strain of the invention in the solution of step a) is between 10⁶ and 10¹² CFU/mL; more preferably the amount of strain of the invention in the solution of step a) is between 10⁶ and 10¹⁰ CFU/mL. The amount of a bacterial strain belonging to the *Gordonibacter* or *Ellagibacter,* or *Slackia* genera in the solution of step a) is not less than 10³ CFU/mL in the solution of step a); preferably not less than 10⁶ CFU/mL in the solution of step a). In a more preferred embodiment, the amount of strain belonging to the *Gordonibacter* or *Ellagibacter* or *Slackia* genera in the solution of step a) is between 10³ and 10¹² CFU/mL; more preferably, the amount of strain belonging to the *Gordonibacter* or *Ellagibacter* or *Slackia* genera in the solution of step a) is between 10⁶ and 10¹² CFU/mL; more preferably the amount of a bacterial strain belonging to the *Gordonibacter* or *Ellagibacter* or *Slackia* genera in the solution of step a) is between 10⁶ and 10¹⁰ CFU/mL. In a preferred embodiment, the strain of the invention and the bacteria belonging to the *Gordonibacter* or *Ellagibacter,* or *Slackia* genera are present in the same proportion. In another preferred embodiment, the strain of the invention and the bacteria belonging to the *Gordonibacter, Ellagibacter,* or *Slackia* genera are present in different relative concentrations leading to varying proportions of the different urolithins of the metabotypes A and/or B.

In a more preferred embodiment, the relative concentrations of the strain of the invention and the bacteria belonging to the *Gordonibacter, Ellagibacter,* or *Slackia* genera is 10:1; more preferably, 100:1.

For the conversion of the first urolithin in step b), the temperature is set at a value between 20 to 50ºC, more preferably between 25 to 45 ºC, and more preferably at 37ºC. The incubation time is at least 1 hour, preferably at least 24 h; and preferably at least 1 week. In the preferred embodiment, the incubation times are 15 h, 72 h, and 1 week leading to varying proportions of the different urolithins of the metabotypes A and/or B.

In another aspect, the invention relates to a pharmaceutical composition, nutritional composition, beverage, dietary supplement, probiotic composition, postbiotic composition, food additive, and/or food that comprises the bacterial strain of the invention in the form of a living or an inactivated cell, or the culture, lysate or culture supernatant of the strain of the invention, the urolithin-producing bacterial consortium of the invention, the urolithin G represented by the formula (I), or a combination of urolithin G represented by the formula (I) and at least one urolithin selected from the list consisting of urolithin M5, urolithin E, urolithin M6, urolithin D, urolithin C, urolithin C, urolithin M7, urolithin A, isourolithin A and urolithin B.

As used herein, the term "pharmaceutical composition" refers to a composition that comprises the bacterial strain of the invention in the form of a living or an inactivated cell; or the culture, lysate, or culture supernatant of the strain of the invention, the urolithin producing bacterial consortium of the invention; or the urolithin G represented by the formula (I); or a combination of urolithin G represented by the formula (I) and at least one urolithin selected from the list consisting of urolithin M5, urolithin E, urolithin M6, urolithin D, urolithin C, urolithin M7, urolithin A, isourolithin A and urolithin B, and at least one pharmaceutically acceptable excipient and/or a carrier.

The term "excipient" refers to a substance that aids in the absorption of any of the components of the composition of the present invention, stabilizes said components, or aids in the preparation of the composition. Thus, the excipients could have the function of keeping the components together, such as starches, sugars, or cellulose, sweetening function, colorant function, drug protection function such as to isolate it from air and/or humidity, function filling of a pill, capsule or any other form of presentation such as dibasic calcium phosphate, disintegrating function to facilitate the dissolution of the components and their absorption in the intestine, without eliminating other types of excipients not mentioned in this paragraph. Therefore, the term "excipient" is defined as that material that, included in the "galenic forms", is added to the active ingredients or their associations to enable their preparation and stability, modify their organoleptic properties or determine the physical-chemical properties of the pharmaceutical or veterinary composition and its bioavailability. Furthermore, the excipient is pharmacologically or veterinary acceptable, that is, the excipient is permitted and evaluated such that it does not cause harm to the organisms to which it is administered.

The "vehicle" or "carrier" is preferably an inert substance. The function of the vehicle is to facilitate the incorporation of other compounds, allow a better dosage and administration, or give consistency and shape to the pharmaceutical composition. Therefore, the vehicle is a substance that is used in the medicine or pharmaceutical or veterinary composition to dilute any of its components to a certain volume or weight; or that, even without diluting said components, it is capable of allowing a better dosage and administration or giving consistency and shape to the medicine or composition. When the presentation form is liquid, the pharmaceutically acceptable carrier is the diluent.

As used in the description, the term "beverage" is used in a broad sense referring to edible beverages suitable for human or animal consumption, such as but not limited to, milk-based beverages or plant-based beverages like soybean-based beverages enriched with ellagic acids or ellagitannins, raspberry, blackberry, cloudberry, boysenberry, strawberry, walnut or geranium-based beverages.

As used in the description, the term "nutritional supplement", "dietary supplement", "nutritional composition", or "nutraceutical" refers to a composition or product in the form of a pill, capsule, tablet, powder, or liquid containing nutrient compounds such as vitamins, minerals, fiber, fatty acids, proteins, amino acids, and (or) other bioactive compounds. Dietary supplements are usually administered to subject human or non-human animals to provide nutrients and/or other bioactive compounds and supplement the diet.

As used in the description, the term "probiotic composition is a composition that contains living microorganisms that are beneficial to the host, and after probiotics are ingested into the human or animal body, they can settle on the intestinal mucosa and provide beneficial effects. Probiotics also can keep people or animals healthy by maintaining natural intestinal microbiota and promoting the formation of healthy metabolites.

As used in the description, the term "postbiotic composition" refers to cell fractions, products, metabolic products, and/or metabolites secreted by live bacteria generated through fermentation to the matrix, or released after bacterial lysis, such as, but not limited to, microbial cell fractions, proteins, enzymes, peptides, teichoic acids, peptidoglycan-derived muropeptides, polysaccharides, extracellular polysaccharides, cell surface proteins, pili-like structures, lipids, and organic acids.

As used in the description, the term "food" is used in a broad sense and covers food for humans as well as food for animals (i.e., feed). In a preferred aspect, the food is for human consumption. The term "food" comprises a "food product," which refers to a product prepared from foods. Non-limiting examples of food products include juices, wines, yogurts, concentrates, jams, jellies, preserves, pastes, and extracts. The terms "food" and "food product" can be used interchangeably.

As used in the description, the term "food additive" is a substance that, without constituting a food or having nutritional value, is intentionally added to foods and beverages in minimal quantities to modify their organoleptic characteristics or facilitate or improve their preparation or conservation process.

In a preferred embodiment, the pharmaceutical composition, nutritional composition, beverage, dietary supplement, probiotic composition, postbiotic composition, food additive, and/or food, the bacterial strain of the invention in the form of a living or an inactivated cell, or the culture, lysate or culture supernatant of the strain of the invention, the urolithin producing bacterial consortium of the invention, the urolithin G represented by the formula (I), or a combination of urolithin G represented by the formula (I) and at least one urolithin selected from the list consisting of urolithin M5, urolithin E, urolithin M6, urolithin D, urolithin C, urolithin M7, urolithin A, isourolithin A and urolithin B, is in the form of a pill, capsule, tablet, powder lyophilized or liquid, wherein it can be administrated to an individual, for example by oral administration.

In a preferred embodiment, the pharmaceutical composition, nutritional composition, beverage, dietary supplement, probiotic composition, food additive, or food, comprises an amount of the strain of the invention or the bacteria of the consortium between 10³ and 10¹¹ cells/day, preferably between 10⁹ and 10¹⁰ cells/day, and more preferably at 10¹⁰ cells/day.

In a preferred embodiment the pharmaceutical composition, nutritional composition, beverage, dietary supplement, probiotic composition, food additive or food, comprises an amount expressed in w/w (respect to the total amount pharmaceutical composition, nutritional composition, etc.) of urolithin G, having the formula (I) or the at least other urolithin of a combination with urolithin G having the formula (I) of at least about 0.0001%, at least about 0.001%, at least about 0.10%, at least about 0.15%, at least about 0.20%, at least about 0.25%, at least about 0.50%, at least about 0.75%, at least about 1%, at least about 10%, at least about 25%, at least about 50%, at least about 75%, at least about 90%, at least about 95%, at least about 99%, at least about 99.99%, no more than about 75%, no more than about 90%, no more than about 95%, no more than about 99%, no more than about 99.99%, from about 0.001% to about 99%, from about 0.001% to about 50%, from about 0.1% to about 99%, from about 1% to about 95%, from about 10% to about 90%, or from about 25% to about 75%.

In a further aspect, the invention relates to a pharmaceutical composition, nutritional composition, beverage, dietary supplement, probiotic composition, postbiotic composition food additive and/or food that comprises the bacterial strain of the invention in the form of a living or an inactivated cell, the culture, lysate or culture supernatant of the strain of the invention, the urolithin producing bacterial consortium of the invention; or the urolithin G represented by the formula (I), or a combination of urolithin G represented by the formula (I) and at least one urolithin selected from the list consisting of urolithin M5, urolithin E, urolithin M6, urolithin D, urolithin C, urolithin M7, urolithin A, isourolithin A and urolithin B, for the use as a medicament (i.e., "drug", "pharmaceutical drug" or "pharmaceutical preparation").

In a preferred embodiment, the pharmaceutical composition comprising the urolithin G represented the formula (I), which can be in the form of, for example, tablets, capsules, pills, powders granulates, suspensions, emulsions, solutions, gels (including hydrogels), pastes, ointments, creams, plasters, drenches, delivery devices, suppositories, enemas, injectables, implants, sprays, aerosols or other suitable forms. Preferably, the pharmaceutical composition comprising the urolithin G represented the formula (I) can be presented in a dosage form suitable for the topical, subcutaneous, intrathecal, intraperitoneal, oral, parenteral, rectal, cutaneous, nasal, vaginal, or ocular administration route. In other embodiments, the pharmaceutical composition can be presented in a dosage form suitable for parenteral administration, mucosal administration, intravenous administration, subcutaneous administration, topical administration, intradermal administration, oral administration, sublingual administration, intranasal administration, or intramuscular administration.

In a further aspect, the invention relates to a pharmaceutical composition, nutritional composition, beverage, dietary supplement, probiotic composition, postbiotic composition, food additive and/or food that comprises the bacterial strain of the invention in the form of a living or an inactivated cell, the culture, lysate or culture supernatant of the strain of the invention, the urolithin producing bacterial consortium of the invention, the urolithin G represented by the formula (I), or a combination of urolithin G represented by the formula (I) and at least one urolithin selected from the list consisting of urolithin M5, urolithin E, urolithin M6, urolithin D, urolithin C, urolithin M7, urolithin A, isourolithin A and urolithin B, for the use in the treatment and/or of human individuals and/or animals with the incapacity of producing urolithins; preferably for use in the treatment and/or prevention of a disease or a condition selected from, but not limited to, muscle disease or a neuromuscular disease, a metabolic disorder, a neurological disorder, and/or cancer; preferably wherein the muscle disease or neuromuscular disease selected from, but not limited to, a myopathy, a muscular dystrophy, Duchenne muscular dystrophy, acute or chronic sarcopenia and muscle atrophy and/or cachexia associated with burns, bed rest, limb immobilization, or major thoracic, abdominal, orthopedic surgery; preferably wherein the neurological disorder or neurodegenerative disease is selected from, but not limited to, Alzheimer's disease or Parkinson's disease; preferably wherein the metabolic disorder is selected from, but not limited to, obesity, *diabetes mellitus,* cardiovascular disease, hyperlipidemia, hypertriglyceridemia, elevated free fatty acids and/or metabolic syndrome; preferably wherein cancer is selected from, but not limited to, a sarcoma, a melanoma, a squamous cell carcinoma of the mouth, throat, larynx, or lung, a genitourinary cancer selected from cervical or bladder cancer, a hematopoietic cancer, a head or neck cancer, a nervous system cancer, prostate cancer, pancreatic cancer, colon cancer, or rectal cancer.

In a further aspect, the invention relates to a urolithin G represented by the formula (I).

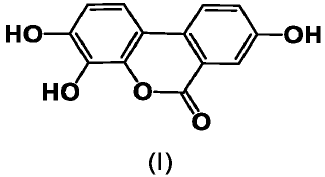

In a further aspect, the invention relates to the urolithin G having the formula (I), or a combination of urolithin G represented by the formula (I) and at least one urolithin selected from the list consisting of urolithin M5, urolithin E, urolithin M6, urolithin D, urolithin C, urolithin M7, urolithin A, isourolithin A and urolithin B, for the use as a medicament (i.e., "drug", "pharmaceutical drug" or "pharmaceutical preparation").

In a further aspect, the invention relates to the urolithin G having the formula (I); or a combination of urolithin G represented by the formula (I) and at least one urolithin selected from the list consisting of urolithin M5, urolithin E, urolithin M6, urolithin D, urolithin C, urolithin M7, urolithin A, isourolithin A and urolithin B; for the use in the treatment and/or prevention of human individuals and/or animals with the incapacity of producing urolithins; preferably for the use in the treatment and/or prevention of a disease or a condition selected from, but not limited to, a muscle disease or a neuromuscular disease, a metabolic disorder, a neurological disorder, metabolic disorder and cancer; preferably wherein the muscle disease or neuromuscular disease is selected from, but not limited to, a myopathy, a muscular dystrophy, Duchenne muscular dystrophy, acute sarcopenia and muscle atrophy and/or cachexia associated with burns, bed rest, limb immobilization, or major thoracic, abdominal, orthopedic surgery; preferably wherein the neurological disorder or neurodegenerative disease is selected from, but not limited to, Alzheimer's disease or Parkinson's disease; preferably wherein the metabolic disorder is selected from, but not limited to, obesity, *diabetes mellitus*, cardiovascular disease, hyperlipidemia, hypertriglyceridemia, elevated free fatty acids and metabolic syndrome; preferably wherein cancer is selected from, but not limited to, a sarcoma, a melanoma, a squamous cell carcinoma of the mouth, throat, larynx, or lung, a genitourinary cancer selected from cervical or bladder cancer, a hematopoietic cancer, a head or neck cancer, a nervous system cancer, prostate cancer, pancreatic cancer, or colon or rectal cancer.

Another aspect of the invention relates to an *in vitro* use of a bacterial strain from the *Enterocloster* genus for the production of the urolithin G, wherein the bacterial strain from the *Enterocloster* genus preferably belongs to any one of the species *Enterocloster bolteae, Enterocloster asparagiformis, Enterocloster citroniae;* more preferably the bacterial strain from the *Enterocloster* genus is selected from any of the species *Enterocloster bolteae DSM15670, Enterocloster bolteae DSM29485, Enterocloster asparagiformis DSM15981* or *Enterocloster citroniae DSM19261.*

In another aspect, the present invention refers to a method for producing urolithin G represented by the formula (I), wherein said method comprises the following steps:
a) Adding the first urolithin as a raw material of urolithin G into a solution containing a bacterial strain from *Enterocloster* genus in the form of a living or an inactivated cell, a culture, a lysate, or culture supernatant of bacterial strain from *Enterocloster* genus,
b) Converting the first urolithin to urolithin G with the bacterial strain from the *Enterocloster* genus in the form of a living or an inactivated cell, a culture, a lysate, or culture supernatant of bacterial strain from the *Enterocloster* genus.

Wherein the first urolithin as the raw material of urolithin G is urolithin D. Preferably the bacterial strain from the *Enterocloster* genus; preferably belongs to any of the species *Enterocloster bolteae*, *Enterocloster asparagiformis*, or *Enterocloster citroniae.*

The term "solution" is understood as it has been described before, being a culture or a solution buffer as defined in the previous method

In a preferred embodiment, the method of producing urolithin G further comprises a step c): purifying and/or concentrating the urolithin G represented by the formula (I) obtained by step b).

In a preferred embodiment, the content of the first urolithin in the solution is usually not less than 0.01 g/L, preferably not less than 0.1 g/L, more preferably not less than 1.0 g/L. On the other hand, the content is usually not more than 100 g/L, preferably not more than 20 g/L, more preferably not more than 10 g/L.

In a preferred embodiment, the amount of bacterial strain from the *Enterocloster* genus in the solution of step a) is not less than 10³ CFU/mL in the solution of step a); preferably not less than 10⁶ CFU/mL in the solution of step a). In a more preferred embodiment, the amount of bacterial strain from *Enterocloster* genus in the solution of step a) is between 10³ and 10¹² CFU/mL; more preferably, the amount of the bacterial strain from *Enterocloster* genus in the solution of step a) is between 10⁶ and 10¹² CFU/mL; more preferably, the amount of the bacterial strain from *Enterocloster* genus in the solution of step a) is between 10⁶ and 10¹⁰ CFU/mL

For the conversion of the first urolithin in step b), the temperature is set at a value between 20 to 50 ºC, preferably between 25 to 45 ºC, and more preferably at 37 ºC. The incubation time is at least 1 hour, preferably at least 24 h, and more preferably at least 1 week.

In a preferred embodiment, the invention methods are performed *in vitro.*

### DESCRIPTION OF THE DRAWINGS

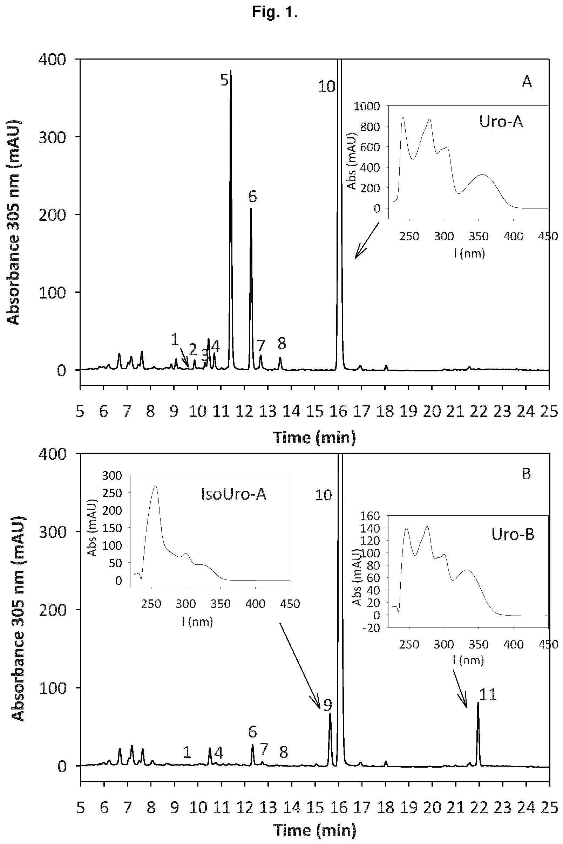

**Figure 1****.** HPLC-DAD chromatogram of *in vitro* metabolism of ellagic acid (EA) by **(A)** *G. urolithinfaciens* DSM 27213 and the strain of the invention, which mimics the urolithin metabotype A (UM-A) and by (B) *E. isourolithinifaciens* DSM 104140^{T} and the strain of the invention *(Enterocloster bolteae* CEBAS S4A9 = DSM 34392) co-culture, which mimics the urolithin metabotype B (UM-B). **1:** Uro-M5; **2:** Uro-D; **3:** Uro-E; **4:** EA; **5:** Uro-M6; **6:** Uro-C; **7:** Uro-G; **8:** Uro-M7; **9:** IsoUro-A; **10:** Uro-A; **11:** Uro-B.

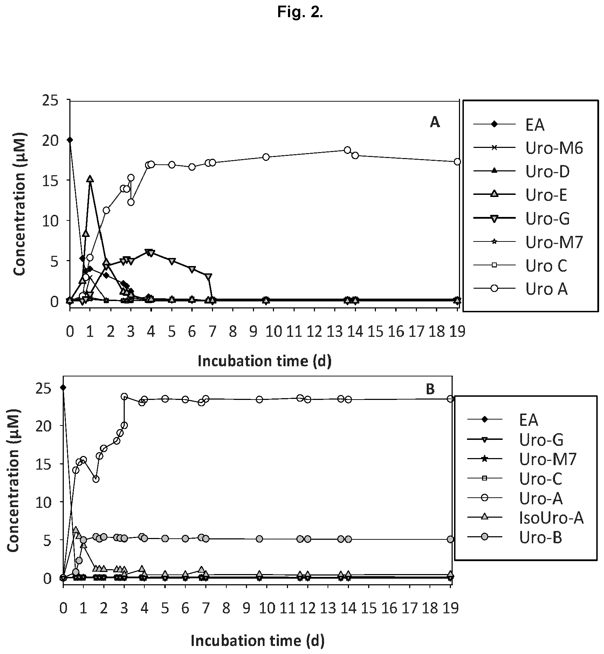

**Figure 2****.** Time-course production of urolithins (Uros) from ellagic acid (EA). **(A)** Metabolism of EA by *G*. *urolithinfaciens* DSM 27213^{T} and the strain of the invention (*Enterocloster bolteae* CEBAS S4A9 = DSM 34392) co-culture. **(B)** Metabolism of EA by *E*. *isourolithinifaciens* DSM 104140 and the strain of the invention co-culture.

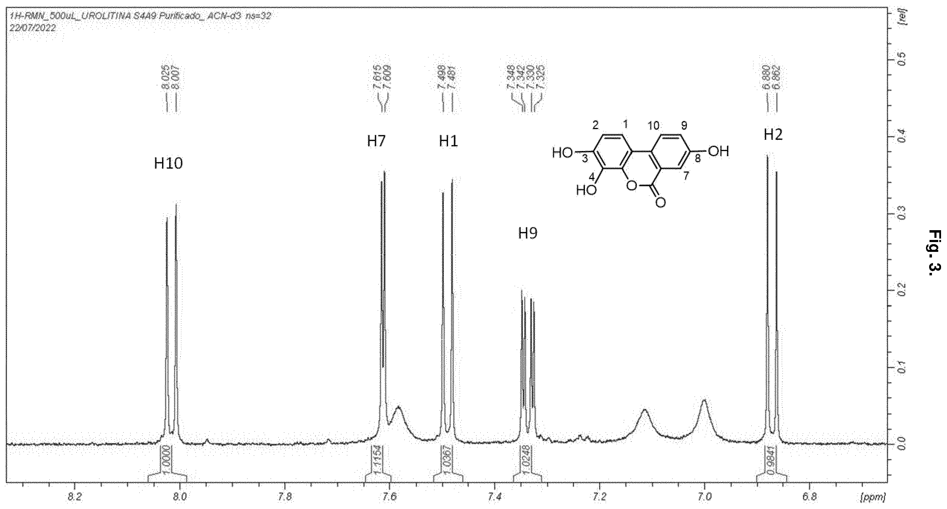

**Figure 3****.** ¹H NMR spectrum of urolithin G (3,4,8,-trihydroxy urolithin).

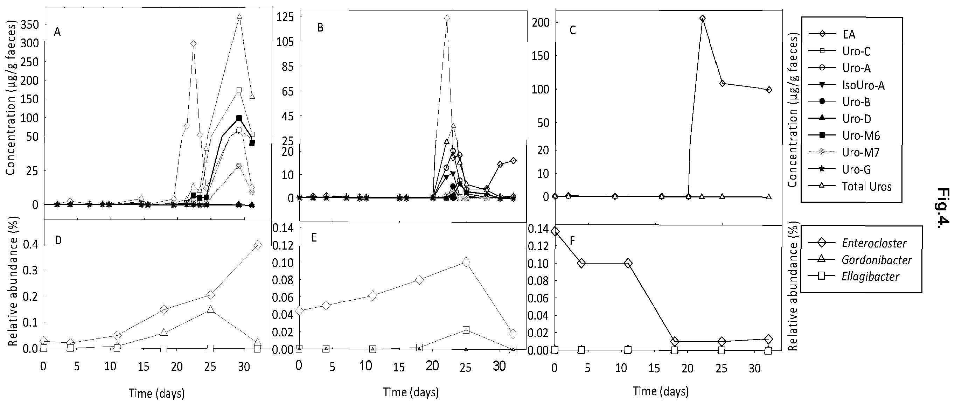

**Figure 4****.** *In vivo* metabolism of EA to urolithins and intestinal colonization of the gut bacterial consortia orally administered to rats from group A (Fig. 4A and Fig. 4D), group B (Fig. 4B and Fig. 4E), and control group (Fig. 4C and Fig. 4F). The results represent the mean of 6 rats (3 males and 3 females) at different time points.

### Examples

### Example 1. Isolation of a new urolithin-producing bacterium from human feces.

A healthy female donor (aged 30), who previously demonstrated to produce Uros *in vivo*, provided the stool samples. After 1/10 (w/v) fecal dilution in nutrient broth (Oxoid, Basingstoke, Hampshire, UK) supplemented with 0.05% L-cysteine hydrochloride (PanReac Química, Barcelona, Spain), the filtrated was homogenized and further diluted in Wilkins-Chalgren anaerobe medium (WAM, Oxoid). The metabolic activity was evaluated by first dissolving 15 µM Uro-C (Dalton Pharma Services) in propylene glycol (PanReac Química SLU, Barcelona, Spain) and then adding it to the broth. After anaerobic incubation, a portion of the culture, having metabolic activity, was seeded on WAM agar. Approximately 200 colonies were collected and inoculated into 5 mL of WAM containing 15 µM Uro-C, and after incubation, their capacity to convert Uro-C was assayed. Uro-C-transforming colonies were sub-cultured until single strains were isolated. The isolation procedure and plate incubation were achieved in an anaerobic chamber (Concept 400, Baker Ruskin Technologies Ltd., Bridgend, South Wales, UK) at 37 ºC. As explained below, samples (5 mL) were prepared for HPLC-DAD-MS analyses of urolithins. Pure bacterial cultures of the strain CEBAS S4A9 were isolated, which showed the capacity to metabolize Uro-C.

The strain CEBAS S4A9 was phylogenetically identified. The almost-complete 16S rRNA gene sequence of the isolated bacterial strain (strain CEBAS S4A9) and the phylogenetic analysis were achieved as previously described (Beltrán et al., 2018, Int. J. Syst. Evol. Microbiol. 68, 1707-1712)

The phylogenetic tree, including the isolated strain CEBAS S4A9 and the most closely related species and known Uros-producing genera (*Gordonibacter* and *Ellagibacter*), were constructed using the Neighbor-joining treeing method as previously described (Selma et al., 2017, Front. Microbiol. 8, 1-8).

The closest relatives of the strain CEBAS S4A9 are *E. bolteae* DSM 15670^{T} (99.8 % 16S rRNA gene sequence similarity), *E. asparagiformis* DSM 15981^{T} (98.0%), *E*. *citroniae* DSM 19261^{T} (97.0%), and *E. clostidioformis* DSM 933^{T} (97.7%). A higher distance was observed with other known Uros-producing bacteria in the phylogenetic tree, i.e., *G. pamelaeae* DSM 19378^{T} (80.9%), *Gordonibacter urolithinfaciens* DSM 27213^{T} (78.2%), and *Ellagibacter isourolithinifaciens* DSM 104140^{T} (80.0%). The comparison of the 16S rRNA gene sequence of the strain showed that the isolate belongs to the *Enterocloster bolteae* species (99.8 % similarity with the type strain E. *bolteae* DSM 15670) from the family Lachnospiraceae. Therefore, the strain CEBAS S4A9 is *Enterocloster bolteae* CEBAS S4A9. The strain *E. bolteae* CEBAS S4A9 was received on September 26^{th}, 2022, for patent deposit according to the Budapest Treaty, at DSMZ - Deutsche Sammlung von Mikroorganismen und Zellkulturen GmbH.

### Example 2. Conversion Testing of EA and Intermediary Uros.

The isolated strain *E. bolteae* CEBAS S4A9 DSM 34392 and representative strains of the closest relatives (*Enterocloster bolteae* DSM 29485, DSM 15670^{T}, *Enterocloster asparagiformis* DSM 15981^{T}, *Enterocloster citroniae* DSM 19261^{T}, *Enterocloster clostidioformis* DSM 933^{T}), obtained from DSMZ culture collection, were used to investigate their capacity to produce final Uros in the presence of EA and other Uro intermediaries. Briefly, isolated and DSMZ strains were separately incubated on the WAM agar plate for 6 days. A single colony was cultivated in a 5 mL WAM tube. Two milliliters of diluted inoculum were transferred to WAM (20 mL), obtaining an initial load of 10⁴ CFU/mL. EA, Uro-M6, Uro-D, Uro-C, Uro-A, IsoUro-A, and Uro-B were dissolved in propylene glycol and added to the 20 mL cultures to obtain a final concentration of 15 µM each.

After incubation in an anoxic environment at 37 ºC, aliquots (5 mL) were taken for HPLC analyses. The HPLC-MS analyses showed that, in contrast to G. *urolithinfaciens* and *E. isourolithinifaciens,* the isolate CEBAS S4A9 or the closest relatives (*E. bolteae* DSM 29485, DSM 15670^{T}, *E. asparagiformis* DSM 15981^{T}, *E. citroniae* DSM 19261^{T}, *E. clostidioformis* DSM 933^{T}) did not metabolize EA (Table 1). However, all the *Enterocloster* species tested, except *E*. *clostidioformis* DSM 933^{T}, metabolized Uro-M6 to other Uros, such as Uro-A, *via* Uro-M7. Table 1 shows the specific Uros produced by each microbial species after incubation with the different precursors. *G. urolithinfaciens* and *E. isourolithinifaciens* also transformed Uro-M6, but *G. urolithinfaciens* rendered Uro-C, whereas *E. isourolithinifaciens* produced IsoUro-A *via* Uro-C. Uro-D was also transformed by most of the *Enterocloster* species tested, rendering a novel trihydroxy urolithin described below and named Urolithin G (Uro-G). In contrast, *G. urolithinfaciens* transformed Uro-D into Uro-C, whereas *E*. *isourolithinifaciens* transformed Uro-D into IsoUro-A *via* Uro-C (Table 1).

**Table 1. Main metabolites produced and the yield (%) after incubating bacterial strains and co-culture with EA and Uros.**

| | **EA** | **Uro-M6** | **Uro-D** | **Uro-C** | **IsoUro-A** |
|---|---|---|---|---|---|
| Strain *E. bolteae* CEBAS S4A9 DSM 34392 | | Uro-M7 (100%) | Uro-G (99.4%) | Uro-A (100%) | Uro-B (99.6%) |
| *E. bolteae* DSM 15670^{T} | | Uro-M7 (100%) | | Uro-A (100%) | |
| *E. bolteae* DSM 29485 | | Uro-M7 (100%) | Uro-G (52%) | Uro-A (100%) | Uro-B (81%) |
| *E. asparagiformis* DSM 15981^{T} | | Uro-A (100%) | Uro-G (48%) | Uro-A (100%) | Uro-B (68%) |
| *E. citroniae* DSM 19261^{T} | | Uro-A (100%) | Uro-G (53%) | Uro-A (100%) | Uro-B (19%) |
| *E. clostidioformis* DSM 933^{T} | | | | | |
| *G. urolithinfaciens* DSM 27213^{T} | Uro-M5, Uro-M6, Uro-C | Uro-C (100%) | Uro-C (54%) | | |
| *E. isourolithinifaciens* DSM 104140^{T} | Uro-M5, Uro-M6, Uro-C, IsoUro-A | Uro-C (100%) | Uro-C (100%) | IsoUro-A (100%) | |
| *S*. *heliotrinireducens* DSM 20476^{T} | | | | IsoUro-A (100%) | |

Most of the *Enterocloster* species tested completely transformed Uro-C into Uro-A except *E*. *clostidioformis* which gave negative reactions for Uros production. *E*. *isourolithinifaciens* and *S*. *heliotrinireducens* also transformed Uro-C, but into IsoUro-A. Unlike *S*. *heliotrinireducens, E. isourolithinifaciens, and G. urolithinfaciens,* the *Enterocloster* species further converted IsoUro-A into Uro-B. Interestingly, the type strain of *E. bolteae* neither transformed IsoUro-A into Uro-B, unlike the other strains of *E. bolteae* tested (CEBAS S4A9 and DSM 29485) (Table 1). None of these bacterial strains metabolized Uro-A or Uro-B (as reported previously, all metabolites were identified by direct comparison (UV spectra and MS) with standards and confirmed by their spectral properties and molecular mass (Garcia-Villalba et al., 2013, J. Agric. Food Chem. 61, 8797-8806).

### Example 3. In Vitro conversion of EA with Gut Bacterial Consortia to Reproduce UMs.

*Gordonibacter urolithinfaciens* DSM 27213^{T}*, Ellagibacter isourolithinifaciens* DSM 104140^{T} obtained from DSMZ culture collection, and *E. bolteae* CEBAS S4A9 DSM 34392 were cultivated anaerobically in 5 mL WAM tubes. First, 2 mL of a diluted aliquot of *G*. *urolithinfaciens* and *E. bolteae* CEBAS S4A9 DSM 34392 (consortium 1) were transferred to WAM (100 mL), obtaining initial loads of 10⁴ CFU/mL. Similarly, 2 mL of diluted aliquots of *E. isourolithinifaciens* and *E. bolteae* CEBAS S4A9 DSM 34392 (consortium 2) were transferred to WAM (100 mL), obtaining initial loads of 10⁴ CFU/mL. Next, EA dissolved in propylene glycol was added to the 100 mL cultures to obtain a final concentration of 25 µM. Then, during incubation in an anoxic environment at 37 ºC, aliquots (5 mL) were taken for HPLC analyses as described below. Incubations were made in triplicate, and the experiment was repeated twice.

For the HPLC-DAD-MS analyses, aliquots (5 mL) collected during the incubation of single and combined bacterial strains were extracted and analyzed by HPLC-DAD-ESI-Q (MS). Briefly, the fermented medium (5 mL) was extracted with ethyl acetate (5 mL) (Labscan, Dublin, Ireland), acidified with 1.5% formic acid (Panreac), vortexed for 2 minutes, and centrifuged at 3500 *g* for 10 min. The organic phase was separated and evaporated, and the dry samples were then re-dissolved in methanol (250 µL) (Romil, Barcelona, Spain). An HPLC system (1200 Series, Agilent Technologies, Madrid, Spain) equipped with a photodiode-array detector (DAD) and a single quadrupole mass spectrometer detector in series (6120 Quadrupole, Agilent Technologies, Madrid, Spain) was used. Calibration curves were obtained for EA, Uro-M6, Uro-D, Uro-C, Uro-A, Uro-B, and IsoUro-A with good linearity (R²>0.998).

The *in vitro* co-culture of *G*. *urolithinfaciens* and *E. bolteae* CEBAS S4A9 DSM 34392 strains (consortium 1) and that of *E*. *isourolithinifaciens* and *E. bolteae* CEBAS S4A9 DSM 34392 strains (consortium 2) were followed to study their Uros-production patterns (Figure 1). HPLC-DAD chromatogram after 15 h incubation showed the production of Uro-M5, Uro-D, Uro-E, Uro-M6, Uro-C, Uro-G, Uro-M7, and Uro-A from EA by the bacterial consortium 1 (potential UM-A reproducer) (Figure 1A). In the case of the bacterial consortium 2 (potential UM-B reproducer), the HPLC-DAD chromatogram showed the production of Uro-M5, Uro-C, Uro-G, Uro-M7, IsoUro-A, Uro-A, and Uro-B from EA (Figure 1B). Uro-E and the novel Uro-G were quantified with the Uro-M7 standard, whereas Uro-M5 was quantified with Uro-M6 as there were no available standards for these urolithins.

When EA was incubated with consortium 1 (potential UM-A reproducer), Uros started to be detected after 15 h incubation (Figure 2A). Uro-M6 and Uro-E (tetrahydroxy-urolithins) also appeared at 15 h with a concentration of 3.34 and 0.21 µM, respectively (Figure 2A). Regarding trihydroxy-urolithins, Uro-C also peaked after 15 h. Uro-M7 started to be detected after 15 h incubation and then progressively decreased. Concerning dihydroxy-urolithins, only Uro-A was detected, reaching a maximum concentration of 18.71 µM (Figure 2A). Most EA disappeared on the third day of incubation, with remaining unmetabolized EA concentrations in the medium lower than 0.06 µM after 5 days (Figure 2A). When EA was incubated with consortium 2 (potential UM-B reproducer), EA started to be converted to Uro-M7 and Uro-C *via* Uro-M5 and Uro-M6. The maximum Uro-M7, Uro-C, and Uro-G concentrations were achieved at 19 h. Then, a plateau was maintained but only in the case of Uro-C. Uro-A started to be detected after 15 h incubation and reached a concentration of 23.4 µM (Figure 2B). Similarly, IsoUro-A and Uro-B started to be detected after 15 h. Most EA was metabolized, and no EA was detected after 7 days (Figure 2B).

### Example 4. Isolation of the New Urolithin (urolithin G) and ¹H NMR Analysis.

During the experiments of example 3, one of the chromatographic peaks, identified as an unknown Uro, was isolated by analytical HPLC with a semipreparative purpose as previously described (Garcia-Villalba et al., 2013, J. Agric. Food Chem. 61, 8797-8806).

The new peaks identified at 305 nm were manually collected. After five injections, the samples collected were taken to dryness in a speed vacuum concentrator, and the residue was re-dissolved in 500 µL of deuterated acetonitrile (ACN-d3) for nuclear magnetic resonance (NMR) analysis as previously described (García-Villalba et al., 2019). The isolated new trihydroxy-urolithin was analyzed by ¹H NMR (500 MHz ACN-d3) with chemical shift values (δ) in ppm: 48 (d; 1H, J_{H1-H2} = 8.2 Hz, H-1); 6.82 (d, 1H, J_{H2-H1} = 8.20 Hz, H-2); 7.61 (d, 1H, J_{H7-H-9} =2.2 Hz, H-7); 7.33 (dd, 1H, J_{H9-H10} = 8.53 Hz, J_{H9-H7} = 2.2 Hz, H-9); 8.03 (d, H, J_{H10-H9}=8.5 Hz, H-10), (Figure 3).

The unknown trihydroxy urolithin ([M - H]⁻ at *m*/*z* 243) showed a Rt at 12.58 min that did not coincide, under the same assay conditions, with the already known trihydroxy urolithins, Uro-C (Rt 12.44 min) and Uro-M7 (Rt 13.59 min) (Figure 1), suggesting a new metabolite (peak 7 in Figures 1A and 1B). UPLC-QTOF-MS analyses showed a molecular formula of C₁₃H₈O₅ with a score of 98.57 and an error of -1.47 for that metabolite. Its MS-MS fragments were similar to those of Uro-M7. The observed NMR spectrum for compound 7 was consistent with the reported spectra of trihydroxy urolithins (García-Villalba et al., 2019, J. Agric. Food Chem. 67, 11099-11107).

### Example 5. In vivo administration of consortia of urolithin-producing bacteria.

*Gordonibacter urolithinfaciens* DSM 27213^{T}, *Ellagibacter isourolithinifaciens* DSM 104140^{T} (1×10⁷ CFU/mL) obtained from DSMZ culture collection, and *E.bolteae* CEBAS S4A9 DSM 34392 (2×10⁹ CFU/mL) were cultivated anaerobically in Wilkins-Chalgren anaerobe medium (WAM, Oxoid) tubes at 37 ºC for 48 h in a Concept 400 anaerobic chamber (Baker Ruskin Technologies Ltd., Bridgend, South Wales, UK) and resuspended in PBS supplemented with 10% of glycerol and 0.05% L-cysteine hydrochloride (PanReac Química, Barcelona, Spain). After anaerobic incubation, consortia were prepared as described before in example 3. Briefly, bacterial consortium A consisted of 1:1 (v/v) of *G. urolithinfaciens* and *E.bolteae* CEBAS S4A9 DSM 34392 cultures; bacterial consortium B consisted of 1:1 (v/v) *E*. *isourolithinifaciens and E.bolteae* CEBAS S4A9 DSM 34392 cultures, and the control cocktail consisted of sterile PBS with 10% glycerol and 0.05% L-cysteine hydrochloride. All cocktails were distributed in aliquots of 3.5 mL and frozen at -80 ºC.

Uros-non-producing Wistar rats (n= 18; female= 9, male= 9) weighing 240 ± 31 g were provided by the Animal Experimentation Centre of the University of Murcia (Spain). Three groups (A, B, and C) were formed by randomly assigning 6 animals each (3 female and 3 male rats) per group. Each rat group was housed in two different cages, dividing animals by sex, in a room with a temperature-controlled environment (22 ± 2 ºC), 55 ± 10% relative humidity, and a controlled light-dark cycle (12h). The rats received a standard chow (Panlab, Barcelona, Spain) containing (%/100g fresh weight) 14.5% proteins, 63.9% carbohydrates and 4% fat (3.2 kcal/g), 4.6% fiber and 13% moisture, supplemented with EA (3.6 mg/100 g of chow). EA was mixed homogenously with ground standard feed and re-pelleted. The EA-enriched diet was stored away from moisture and light. The EA dose given to the rats in the diet was approximately 0.72 mg/rat/day diet (EA 1x diet), equivalent to 41 mg/human/day following the HED (human equivalent dose) formula. All groups were fed the EA 1x diet and tap water ad libitum throughout the experiment (6 weeks). At week 3, an extra dose of 1.5 mg EA/rat/day dissolved in water was oral-gavaged to the animals (EA 3x diet). Finally, at week 4, animals returned to the EA 1x diet plus ≈ 5 g walnuts per rat/day, and this was maintained until the end of the study. Weight and food and water intake were measured every week.

Group A received oral gavage of 500 µL of bacterial consortium A. Group B received 500 µL of bacterial consortium B, and Group C (control) received 500 µL of PBS. Oral gavages were performed every 2 days during the first two weeks and every weekday during the next 2 weeks. The last week (from day 28 to 35) was a washout week, and the animals were not given oral gavage. Animals were sacrificed with carbon dioxide, and organs were collected and weighed after sacrifice.

Fecal samples (0.2-0.5 g) were extracted, in a proportion of 1:10, with a solution of MeOH/H₂O (80/20), acidified with 0.1% HCI and homogenized by vortexing for 2 min and shaking them at 1500 rpm at room temperature for 10 min in a thermoblock. The suspension was centrifugated at 14,000 *g* at 4 °C for 10 min, and the supernatant was filtered through a 0.22 µm PVDF membrane filter (Millipore Corp., Bedford, MA) and diluted 3x with MeOH (0.1% formic acid) before injection in UPLC-ESI-QTOF-MS system. A UPLC system (Agilent 1290 Infinity) coupled to a Quadrupole time-of-flight (QTOF) (6550 Accurate-Mass) (Agilent Technologies, Waldbronn, Germany) was used to analyze the fecal samples for metabolites detection. Briefly, a Poroshell 120 EC-C18 reverse-phase column was used for metabolite separation using a gradient mode with HPLC-grade water as mobile phase A and acetonitrile as mobile phase B, both acidified with 0.1% formic acid. The flow rate was 0.4 mL/min, and 5 µL the injection volume. MassHunter Qualitative Analysis software (version B.10, Agilent Technologies, Waldbronn, Germany) was used to process the data.

Data are expressed as mean ± standard deviation (SD). Statistical analysis was performed with SPSS Software version 27.0 (SPSS Inc., Chicago, IL, USA), and differences with p<0.05 were considered statistically significant. Data normality was evaluated with the Shapiro-Wilk test. Comparisons between different treatment groups were carried out using repeated measured analysis of variance (ANOVA) with Bonferroni's or Dunnett's T3 post hoc test, depending on whether the data presented a normal or non-normal distribution, respectively. Pearson correlation was used to analyze possible associations between variables when data distribution was normal, while Spearman correlation was applied in non-normal data distribution. Data plots were performed using Sigma Plot 14.5 (Systat Software, San Jose, CA, USA).

The Uro analysis in feces showed that initially (T0), the rats did not produce Uros after ingesting EA powder (Figures 4A, 4B, 4C). However, after administering the bacterial consortia A (Figure 4A) and B (Figure 4B), rats became capable of producing Uros. During the first three weeks, when the rats from groups A and B only ingested EA powder, the detection of Uros (range: 0-1.43 µg/g) and their EA precursor (range: 0.07-4.54 µg/g) in feces was low. However, from day 21, when the consumption of walnuts started as a second EA source, there was a very significant increase in fecal EA (Figures 4A, 4B, 4C) and Uros production in group A (Figure 4A) and B (Figure 4B). Moreover, differences in the Uro profile were observed between groups A and B. In group A, fecal Uro-C, Uro-M6, Uro-M7, and the final metabolite Uro-A were predominant. In contrast, in group B, the three final Uros (Uro-A, Uro-B, and IsoUro-A) were present, whereas the intermediates were scarce. However, a higher fecal concentration of the recently described Uro-G was detected in group B than in group A. In addition, the Uro-B concentration was lower than Uro-A and IsoUro-A in group B. Furthermore, Uro-A and IsoUro-A, but not Uro-B, were produced in all rats of group B (Figure 4B). When walnuts were added to the diet of control rats (group C) on day 21, a higher fecal concentration of EA was also observed than before walnut consumption, where fecal EA concentration was scant (Figure 4C). However, neither EA powder intake nor walnut consumption promoted the production of Uros in the gut of control rats (i.e., with no bacterial consortia).

The levels of Uros-producing bacteria in the fecal samples of rats were also analyzed. DNA extraction from fecal samples was performed with the NucleoSpin^{®} Tissue DNA Purification Kit (Macherey-Nagel, Germany). DNA was then quantified through Fluorimetry (Qubit 3.0 - ThermoFisher ScientificTM, UK) and Spectrophotometry (NanoDrop - ThermoFisher ScientificTM, UK). The primers and probe designed for detecting and quantifying *Ellagibacter* and *Enterocloster* are shown in Table 2. RTi-PCR was performed using an ABI 7500 sequence detection system. Final concentrations of each primer and probe of 300 and 375nM for the *Ellagibacter* genus and 200 and 500 nM for the *Enterocloster* genus, respectively, were used. A conventional RTi-PCR protocol was carried out for the DNA amplification of the *Enterocloster* genus. The ramping profile was 1 cycle at 95 °C for 5 min, followed by 40 cycles of 95 °C for 15 s, 65 °C for 40 s, and 72 °C for 31 s. Finally, 1 cycle at 72 °C for 5 min was added.

**Table 2. TaqMan primers and probe for detecting and quantifying the Gordonibacter genus and the novel designed sets for detecting and quantifying the Ellagibacter and Enterocloster genera.**

| **Primer** | **SEQ ID** | **Oligonucleotide sequence 5'-3'** |
|---|---|---|
| *Gordonibacter* Forward | SEQ ID NO.1 | GGCTCGAGTTTGGTAGAGGAAGAT |
| *Gordonibacter* Reverse | SEQ ID NO.2 | GGCCCAGAAGACTGCCTT |
| *Gordonibacter* Probe | SEQ ID NO.3 | 6FAM-AATTCCCGGTGTAGCGGTGGAATGC-BBQ |
| *Ellagibacter* Forward | SEQ ID NO.4 | GCTAGGTGTGGGGAAAC |
| *Ellagibacter* Reverse | SEQ ID NO.5 | CTCAAAGGAATTGACGG |
| *Ellagibacter* Probe | SEQ ID NO.6 | 6FAM-TACGGCGGCAACGC-BBQ |
| *Enterocloster* Forward | SEQ ID NO.7 | ACGTCCCAGTTCGGACTGTA |
| *Enterocloster* Reverse | SEQ ID NO.8 | GTTGCTGACTCCCATGGTGT |
| *Enterocloster* Probe | SEQ ID NO.9 | 6FAM-CAACCCGACTACACGAAGCTGGAA-BBQ |

| | | |
|---|---|---|
| ^{a} 16S ribosomal RNA gene. | | |

Initially, before the rats of groups A and B started the consumption of the bacterial cocktails A and B, respectively, the *Gordonibacter* or *Ellagibacter* genera were under the limit of detection in the fecal samples (Figures 4D, 4E). However, the *Enterocloster* genus was detected in the rat feces from all groups (A, B, and C) at baseline (Figures 4D, 4E, and 4F). The relative abundance (mean±S.D.) of the *Enterocloster* genus in groups A, B, and C was 0.03±0.02, 0.04±0.06 and 0.13±0.17, respectively, but without significant differences (p=0.570). The consumption of the bacterial consortia A (*Gordonibacter* and *Enterocloster* genera) and B (*Ellagibacter* and *Enterocloster* genera) in rat groups A and B resulted in the appearance and gradual increase of the *Gordonibacter* (Figure 4D) and *Ellagibacter* (Figure 4E), respectively, at one time-point. The fecal *Enterocloster* levels also increased throughout the study in groups A and B (Figures 4D, 4E). However, a reduced concentration of the administered bacteria was detected 1 week after stopping the consumption of the bacterial cocktails (end time of the study), which resulted in a significant decrease in the production of Uros too (Figure 4). In the control group, the *Ellagibacter* genus was under the limit of detection throughout the study, and the *Enterocloster* and *Gordonibacter* genera remained at very low levels (Figure 4F).

Potential side effects from ingestion of the bacterial consortia were investigated. No adverse effects on growth, food intake, and vital organs were observed after 4 weeks of oral administration by gavage of bacterial consortia in rats from groups A and B compared to the control group. An analysis of the hematological variables was performed (number of platelets, hematocrit, erythrocytes, hemoglobin, neutrophils, lymphocytes, monocytes, eosinophils, basophils, Mean Corpuscular Volume, Mean Corpuscular Hemoglobin, Mean Corpuscular Hemoglobin Concentration, Erythrocyte Distribution, Mean Platelet Volume, Plateletcrit, Platelet Distribution Width, Mean Platelet Component, Mean Platelet Mass, Platelet Count, Reticulocyte Haemoglobin Content, and Mean Reticulocyte Corpuscular Volume, observing some differences between males and females in some variables, such as erythrocyte levels (p=0.05) (females control: 6.8±0.8, females group A: 7.2±1.2, females group B: 7.2±0.4, males control: 6.3±0.5, males group A: 7.6±1.3, males group B: 7.7±0.2, values expressed as ×10⁶ cells/µL), red blood cell distribution index (RDW) (p=0.005) (females control: 10.6±0.3, females group A: 11.1±0.7, females group B: 10.3±0.5, males control: 11.2±0.2, males group A: 11.5±0.2, males group B: 11.4±0.5, values expressed as %), and the number of leukocytes (p=0.006) (females control: 3.0±0.6, females group A: 4.0±0.9, females group B: 4.9±2.0, males control: 5.7±0.8, males group A: 7.3±0.9, males group B: 4.9±0.4, values expressed as ×10³ cells/µL). However, no differences were observed for any variables between groups that consumed the bacterial consortia (groups A and B) and the control group (group C). The serobiochemical values were also analyzed (Total Proteins, Albumin; Globulin, Creatinine, Glucose, Cholesterol, Triglycerides, Calcium, Phosphorus, Alkaline Phosphatase, Gamma Glutamyl Transpeptidase, Aspartate Aminotransferase, Alanine Aminotransferase etc) and differences were also detected only between males and females in some variables such as creatinine index (females control: 0.6±0.0, females group A: 0.7±0.0, females group B: 0.6±0.0, males control: 0.6±0.0, males group A: 0.6±0.0, males group B 0.6±0.0, values expressed as mg/dL, p<0.001), phosphorus (females control: 7.5±1.6, females group A: 10.4±0.9, females group B: 8.1±2.1, males control: 10.0±1.9, males group A: 10.3±2.1, males group B: 11.5±3.0, values expressed as mg/dL, p=0.007), alkaline phosphatase (ALP) (females control: 207.2±131.5, females group A: 236.2±60.6, females group B: 321.5±71.8, males control: 453.9±158.3, males group A: 360.7±52.5, males group B: 497.3±116.0, values expressed as UI/L p=0.003), and thyroxine (T4) (females control: 3.6±1.1, females group A: 3.6±0.2, females group B: 4.1±0.9, males control: 3.7±1.6, males group A: 4.0±0.3, males group B: 4.4±0.7, values expressed as µg/dL, p=0.002). However, no differences were observed for any variable between the groups that consumed the bacterial cocktails (groups A and B) and the control group (group C).

## Claims

1. A bacterial strain belonging to the *Enterocloster* genus that produces urolithins, wherein said strain is *Enterocloster bolteae* CEBAS S4A9 DSM 34392 in the form of a living or an inactivated cell.

2. A culture, a lysate, or a culture supernatant of the bacterial strain, according to claim 1.

3. The *in vitro* use of the bacterial strain, according to claim 1, or the culture, lysate, or culture supernatant, according to claim 2, for producing urolithins.

4. The use, according to claim 3, wherein the production of urolithins is carried out starting from a first urolithin as raw material, wherein the first urolithin is urolithin M5 and the produced urolithin is urolithin D or E; or the first urolithin is urolithin M6 and the produced urolithin is M7; or the first urolithin is D and the produced urolithin is G; or the first urolithin is C, and the produced urolithin is A, or the first urolithin is isourolithin A and the produced urolithin is urolithin B; wherein the urolithin G is a urolithin represented by the formula (I):

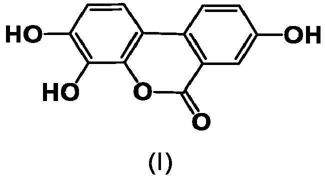

5. A urolithin-producing bacterial consortium that comprises:
a. The bacterial strain, according to claim 1, or the culture, lysate, or culture supernatant, according to claim 2 and,
b. At least another living or inactivated bacterial strain belonging to the *Gordonibacter* and/or *Ellagibacter* and/or *Slackia* genera; or a culture, lysate, or culture supernatant of a bacterium belonging to the *Gordonibacter* and/or *Ellagibacter* and/or *Slackia* genera.

6. The urolithin-producing bacterial consortium, according to claim 5, wherein:
- The bacterial strain belonging to the *Gordonibacter* genus is a bacterium belonging to *Gordonibacter urolithinfaciens*, *Gordonibacter pamelaeae*, and/or *Gordonibacter massiliensis*; preferably *Gordonibacter urolithinfaciens* DSM27213, *Gordonibacter pamelaeae* DSM19378, or *Gordonibacter* 28C; and/or,
- The bacterial strain belonging to the *Ellagibacter* genus is a bacterium belonging to *Ellagibacter isourolithinifaciens;* preferably *Ellagibacter isourolithinifaciens* DSM 104140; and/or,
- The bacterial strain belonging to the *Slackia* genus is a bacterium belonging to *Slackia heliotrinireducens;* preferably *Slackia heliotrinireducens* DSM 20476.

7. The *in vitro* use of the urolithin-producing bacterial consortium according to any of claims 5 to 6 for the production of urolithins from a raw material, preferably wherein the raw material comprises urolithin M5, ellagic acid, and/or ellagitannins.

8. The use, according to claim 7, wherein the produced urolithins belong to the metabotype A when the urolithin-producing bacterial consortium comprises a) the bacterial strain according to claim 1 or the culture, lysate or culture supernatant, according to claim 2, and b) at least a living or inactivated bacterial strain belonging to the *Gordonibacter* genus or a culture, lysate or culture supernatant of the bacterium belonging to the *Gordonibacter* genus; wherein the produced urolithin of the metabotype A are urolithin D, urolithin E, urolithin M6, urolithin C, urolithin G, urolithin M7 and/or urolithin A.

9. The use, according to claim 7, wherein the produced urolithins belong to the metabotype B when the urolithin-producing bacterial consortium comprises a) the bacterial strain, according to claim 1, or the culture, lysate, or culture supernatant, according to claim 2, and b) at least a living or inactivated bacterial strain belonging to the *Ellagibacter* genus or a culture, lysate or culture supernatant of the bacterium belonging to the *Ellagibacter* genus, and optionally c) at least a living or inactivated bacterial strain belonging to the *Slackia* genus or a culture, lysate or culture supernatant of the bacterium belonging to the *Slackia* genus; wherein the produced urolithins of the metabotype B are urolithin C, urolithin G, urolithin M7, isourolithin A, urolithin A and/or urolithin B.

10. The use, according to claim 7, wherein the produced urolithins belong to the metabotype B when the urolithin-producing bacterial consortium comprises a) the bacterial strain, according to claim 1, or the culture, lysate, or culture supernatant, according to claim 2, and b) at least a living or inactivated bacterial strain belonging to the *Gordonibacter* genus or a culture, lysate or culture supernatant of these bacteria, and c) at least a living or inactivated bacterial strain belonging to the *Slackia* genus or a culture, lysate or culture supernatant of the bacterium belonging to the *Slackia* genus; wherein the produced urolithins of the metabotype B are urolithin C, urolithin G, urolithin M7, isourolithin A, urolithin A and/or urolithin B.

11. A pharmaceutical composition, nutritional composition, beverage, dietary supplement, probiotic composition, postbiotic composition, food additive, and/or food that comprises the bacterial strain, according to claim 1; or the culture, lysate, or culture supernatant, according to claim 2, the urolithin producing bacterial consortium according to any of claims 5 to 6, the urolithin G represented by the formula (I) according to claim 14, or a combination of urolithin G represented by the formula (I) according to claim 14 and at least one urolithin selected from the list consisting of urolithin M5, urolithin E, urolithin M6, urolithin D, urolithin C, urolithin M7, urolithin A, isourolithin A and/or urolithin B.

12. The bacterial strain, according to claim 1, the culture, lysate or culture supernatant, according to claim 2, the urolithin-producing bacterial consortium according to any of claims 5 to 6, the pharmaceutical composition, nutritional composition, beverage, dietary supplement, probiotic composition, postbiotic composition, food additive and/or food, according to claim 11, the urolithin G represented by the formula (I), according to claim 14, or a combination of urolithin G represented by the formula (I) according to claim 14 and at least one urolithin selected from the list consisting of urolithin M5, urolithin E, urolithin M6, urolithin D, urolithin C, urolithin M7, urolithin A, isourolithin A and urolithin B, for the use as a medicament.

13. The bacterial strain, according to claim 1, the culture, lysate or culture supernatant, according to claim 2, the urolithin producing bacterial consortium according to any of claims 5 to 6; or the pharmaceutical composition, nutritional composition, beverage, dietary supplement, probiotic composition, postbiotic composition food additive and/or food, according to claim 11, for the use in the treatment of human individuals and/or animals with the incapacity of producing urolithins; preferably for use in the treatment and/or prevention of a disease or a condition selected from a muscle disease or a neuromuscular disease, a metabolic disorder, a neurological disorder, metabolic disorder and cancer; preferably wherein the muscle disease or neuromuscular disease is selected from a myopathy, a muscular dystrophy, Duchenne muscular dystrophy, acute sarcopenia and muscle atrophy and/or cachexia associated with burns, bed rest, limb immobilization, or major thoracic, abdominal, orthopedic surgery; preferably wherein the neurological disorder or neurodegenerative disease is selected from Alzheimer's disease or Parkinson's disease; preferably wherein the metabolic disorder is selected from obesity, *diabetes mellitus*, cardiovascular disease, hyperlipidemia, hypertriglyceridemia, elevated free fatty acids or metabolic syndrome; preferably wherein cancer is selected from a sarcoma, a melanoma, a squamous cell carcinoma of the mouth, throat, larynx, or lung, a genitourinary cancer selected from cervical or bladder cancer, a hematopoietic cancer, a head or neck cancer, a nervous system cancer, prostate cancer, pancreatic cancer, and colon or rectal cancer.

14. Urolithin G, represented by the formula (I):

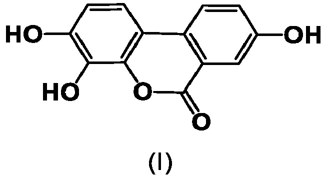

15. The urolithin G, according to claim 14, or a combination of urolithin G represented by the formula (I) according to claim 14 and at least one urolithin selected from the list consisting of urolithin M5, urolithin E, urolithin M6, urolithin D, urolithin C, urolithin C, urolithin M7, urolithin A, isourolithin A and urolithin B, for the use in the treatment and/or prevention of a disease or a condition selected from muscle disease or a neuromuscular disease, a metabolic disorder, a neurological disorder, metabolic disorder or cancer; preferably wherein the muscle disease or neuromuscular disease is selected form myopathy, a muscular dystrophy, Duchenne muscular dystrophy, acute sarcopenia or muscle atrophy and/or cachexia associated with burns, bed rest, limb immobilization, or major thoracic, abdominal, orthopedic surgery; preferably wherein the neurological disorder or neurodegenerative disease is selected from Alzheimer's disease or Parkinson's disease; preferably wherein the metabolic disorder is selected from obesity, diabetes mellitus, cardiovascular disease, hyperlipidemia, hypertriglyceridemia, elevated free fatty acids or metabolic syndrome; preferably wherein cancer is selected from a sarcoma, a melanoma, a squamous cell carcinoma of the mouth, throat, larynx, or lung, a genitourinary cancer selected from cervical or bladder cancer, a hematopoietic cancer, a head and neck cancer, a nervous system cancer, prostate cancer, pancreatic cancer or colon cancer.
